# EUROPEAN PATENT APPLICATION

(11) **EP 1 358 888 A1**
(43) Date of publication of application: **05.11.2003**
(21) Application number: 03004306.1
(22) Date of filing: 27.02.2003
(51) Int. Cl.: A61K 38/17, A61K 39/395, A61P 9/10, A61P 17/02, A61P 35/00

(54) **The human peptide antibiotic LL-37/hCAP-18 is an inducer of angiogenesis**

(30) Priority: 28.02.2002 EP 02004656
(71) Applicant: Bals, Robert, 35043 Marburg (DE); Koczulla, Andreas Rembert, 35043 Marburg (DE); von Degenfeld, Georges, Los Altos Hills, CA 94022 (US)
(72) Inventor: Bals, Robert, 35043 Marburg (DE); Koczulla, Andreas Rembert, 35043 Marburg (DE); von Degenfeld, Georges, Los Altos Hills, CA 94022 (US)
(74) Representative: VOSSIUS & PARTNER

(57) **Abstract**

The present invention relates to the use of the peptide LL-37 for the preparation of a pharmaceutical composition for the prevention or treatment of a disease caused by or resulting in a reduced level of angiogenesis or of arteriogenesis, or for the treatment of (infected) wounds or cancer. Preferably, said disease is a disease associated with reduced blood flow. It is most preferred that said disease is atherosclerosis, coronary heart disease, stroke, arterial occlusive disease or an ulcer.

## Description

The present invention relates to the use of the peptide LL-37 for the preparation of a pharmaceutical composition for the prevention or treatment of a disease caused by or resulting in a reduced level of angiogenesis or of arteriogenesis, or for the treatment of (infected) wounds or the inhibition of tumor growth. Preferably, said disease is a disease associated with reduced or increased blood flow. It is most preferred that said disease is atherosclerosis, coronary heart disease, stroke, arterial occlusive disease, ulcer, defective wound healing or cancer.

A variety of documents is cited in this specification. The disclosure content of these documents, including manufacturer's manuals, is herewith incorporated by reference.

Angiogenesis is a central physiological and pathological process and is involved in a variety of diseases. The formation of new blood vessels is a prerequisite of tissue repair and wound healing 1,2. The proliferation of endothelial cells and the sprouting of new vessels are important components of angiogenesis. Cellular components of angiogenesis are endothelial, smooth muscle, connective tissue, and inflammatory cells. Blood vessel formation is initiated by various factors ranging from mechanical stress and hypoxia to the presence of soluble angiogenic mediators 2 and involves the sprouting of small capillaries (angiogenesis) and the growth of preexisting vessels (arteriogenesis). Angiogenesis is induced by soluble mediators such as fibroblast growth factor (FGF) or vascular endothelium growth factor (VEGF) and by stem or progenitor cells.

Angiogenesis is a prerequisite for:
- the **formation of organs and tissues** during the development and the regeneration.
- **wound healing** including tissue regeneration after injury. Defective wound healing is involved in several human diseases such as ulcers (ischemic, veno occlusive, diabetes).
- **Adaptation to ischemia** e.g. after myocardial, cerebral, pulmonary or gastrointestinal infarction.
- **Atherosclerosis** is a pathological degenerative process that is associated with angiogenesis as compensatory mechanism. Arteriosclerosis contributes to important human diseases such as coronary heart diseases and stroke.
- **Cancer growth** involves the formation of new vessels to supply the tumor with oxygen and nutrients.

Angiogenesis is a focus of therapeutic approaches. The aim of these approaches is to modify the course of different diseases by the promotion or inhibition of angiogenesis. The term "therapeutic angiogenesis" describes an approach to stimulate growth of vessels by various means. Different strategies have been used to achieve this goal, for example the application of different angiogenic factors such as VEGF.

In addition, it has been shown by in vitro and in vivo studies that a porcine member of the cathelicidins, a family of antimicrobial peptides, is implicated in angiogenesis. The active porcine peptide termed PR-39 is obtained after cleavage of a propeptide and consists of the C-terminal 39 amino acids thereof. It has a high content of the amino acids proline and arginine, is taken up by cells and mediates its angiogenic activity by a modulatory activity on the cellular proteasome.

A human homologue of PR-39 has not been identified. Whereas the prior art has certainly contributed to the understanding of the molecular basis of angiogenesis, prior art approaches suffer from induction of an antibody response to non-human peptides. Thus, there remains a need in the art to devise appropriate approaches for therapeutic angiogenesis in humans. The solution to said technical problem is achieved by providing the embodiments characterized in the claims.

Accordingly, the present invention relates to use of a peptide having the amino acid sequence LLGDFFRKSKEKIGKEFKRIVQRIKDFLRNLVPRTES (also termed LL-37) for the preparation of a pharmaceutical composition for the prevention or treatment of a disease caused by or resulting in a reduced level of angiogenesis or of arteriogenesis, or for the treatment of wounds.

The term "pharmaceutical composition", as used in accordance with the present invention, comprises at least the compound as identified herein above, namely LL-37. It may further comprise molecules which are capable of optimizing the therapeutic process , e.g. antibiotic substances for the treatment of infections and/or other angiogenic substances for enhancing angiogenesis.

In accordance with the present invention, it was found that endothelial cells express a molecule called formyl peptide receptor-like 1 (FPRL1). This receptor is known to bind to LL-37, a human antimicrobial peptide. Antimicrobial peptides are effector molecules of the innate immune system with microbicidal and proinflammatory activities. LL-37 is also called hCAP-18. LL-37 or LL-37/hCAP-18 refer to the 37 C-terminal amino acid residues of the peptide. LL-37/hCAP-18 is the only antimicrobial peptide of the cathelicidin family identified from humans. It is expressed in leukocytes such as neutrophils, monocytes, NK cells, T cells, and B cells. In addition, LL-37/hCAP-18 is secreted from epithelial cells of the testis, skin 3,4, the gastrointestinal 5 and the respiratory tract 5. LL-37/hCAP-18 has been identified in the airway surface fluid 5,6 and in wound fluid 3.

Surprisingly, it was found in accordance with the present invention that the human antimicrobial peptide LL-37/hCAP-18 is capable to induce functionally important angiogenesis in cell culture and in vivo by activation of the receptor molecule FPRL1. The term angiogenesis used within the meaning of the invention comprises the sprouting of capillaries and the growth of collaterals ("arteriogenesis"). The mechanism of angiogenic activity of LL-37/hCAP-18 involves binding to FPRL1, a G-protein coupled receptor, with subsequent activation of intracellular signaling. This mechanism differs from the mode of action described for PR-39, a proline-arginine rich, porcine cathelicidin antimicrobial peptide that has been shown to modulate angiogenesis by inhibition of the ubiquitin-proteasome dependent degradation of HIF-1α ¹⁴. In addition, the primary amino acid structure of PR-39 is totally different from the struture of LL-37. Accordingly, the finding forming the basis of the present invention was highly surprising even in view of the prior art knowledge on PR-39.

The present invention further relates to the use of an agent which suppresses angiogenesis through the inhibition of the biological activity of a LL-37 for the preparation of a pharmaceutical composition for the treatment of tumors. Such agent can be used accordingly, to block the growth of collateral arteries and/or other arteries from preexisting arteriolar connections.

In accordance with the present invention, said agent blocks an interaction of LL-37 and its receptor. In accordance with the present invention said agent inhibits the biological activity of LL-37 or blocks an intracellular signal or signal cascade mediated by an LL-37 specific receptor or receptor molecule.

Preferably, said receptor is FPRL1.

Said receptor or specific domains thereof which are responsible for triggering a signal leading to angiogenesis may be blocked or modulated by methods described herein.

In a preferred embodiment, said agent is a(n) antibody, polypeptide, peptide, nucleic acid, small organic compound, ligand, hormone, PNA or peptidomimetic.
Nucleic acid molecules specifically hybridizing to LL-37 or its receptor encoding genes and/or their regulatory sequences may be used for repression of expression of said gene by methods as disclosed in e.g. PCT/DE00/00244, PCT/US98/27233 or PCT/US01/10188, or for example due to an antisense or triple helix effect or they may be used for the construction of appropriate ribozymes (see, e.g., EP-B1 0 291 533, EP-A1 0 321 201, EP-A2 0 360 257) which specifically cleave the (pre)-mRNA of a gene encoding LL-37. The nucleic and amino acid sequences encoding LL-37 are known in the art and described, for example, in GeneBank, accession number: Protein P49913, mRNA NM_004345, gene X96735. Selection of appropriate target sites and corresponding ribozymes can be done as described for example in Steinecke, Ribozymes, Methods in Cell Biology 50, Galbraith et al. eds Academic Press, Inc. (1995), 449-460.

Nucleic acids comprise DNA or RNA or hybrids thereof. Furthermore, said nucleic acid may contain, for example, thioester bonds and/or nucleotide analogues, commonly used in oligonucleotide anti-sense approaches. Said modifications may be useful for the stabilization of the nucleic acid molecule against endo- and/or exonucleases in the cell. Furthermore, the so-called "peptide nucleic acid" (PNA) technique can be used for the inhibition of the expression of a gene encoding LL-37 or FPRL1. For example, the binding of PNAs to complementary as well as various single stranded RNA and DNA nucleic acid molecules can be systematically investigated using, e.g., thermal denaturation and BIAcore surface-interaction techniques (Jensen, Biochemistry 36 (1997), 5072-5077). The synthesis of PNAs can be performed according to methods known in the art, for example, as described in Koch, J. Pept. Res. 49 (1997), 80-88; Finn, Nucleic Acids Research 24 (1996), 3357-3363. Furthermore, folding simulations and computer redesign of structural motifs of LL-37 and its receptor(s), e.g. FPRL1 can be performed as described for the design of compounds or drugs capable of inhibiting the biological activity of LL-37.
Furthermore, antibodies may be employed specifically recognizing LL-37 or its receptor(s) or parts, i.e. specific fragments or epitopes, of such LL-37 and receptors thereby inactivating the LL-37 peptide or the LL-37 receptor. These antibodies can be monoclonal antibodies, polyclonal antibodies or synthetic antibodies as well as fragments of antibodies, such as Fab, Fv or scFv fragments etc. Antibodies or fragments thereof can be obtained by using methods which are described, e.g., in Harlow and Lane "Antibodies, A Laboratory Manual", CSH Press, Cold Spring Harbor, 1988 or EP-B1 0 451 216 and references cited therein, e.g. by immunization of experimental animals, optional in combination with the hybridoma technique. Furthermore, surface plasmon resonance as employed in the BIAcore system can be used to increase the efficiency of phage antibodies which bind to an epitope of LL-37 or its receptor (Schier, Human Antibodies Hybridomas 7 (1996), 97-105; Malmborg, J. Immunol. Methods 183 (1995), 7-13).
Putative inhibitors which can be used in accordance with the present invention capable of inhibiting the biological activity of a LL-37 or its receptor may be further identified according to the methods known in the art, for example as described in EP-A-0 403 506 or in the appended examples.

In a most preferred embodiment, the agent which blocks the interaction of the LL-37 and its receptor is selected from the group consisting of
(i) an anti-LL-37 antibody and an anti-LL-37-receptor antibody; and/or
(ii) a non-stimulatory form of LL-37 or a soluble form of a LL-37-receptor.

The present invention further encompasses that the agent is designed to be expressed and/or applied in vascular cells or cells surrounding preexisting arteriolar connections to a tumor.

In a preferred embodiment the tumor is preferably selected from the group consisting of carcinoma or sarcoma including cancer of the bile duct, brain, breast, colon, stomach, male and female reproductive organs, lung and airways, skin, gallbladder, liver, nasopharynx, nerve cells, kidney, prostate, glands and Kaposi's sarcoma.

Preferably, the pharmaceutical composition is administered or for administration to a mammal, more preferred to a primate and most preferred to a human.

The pharmaceutical composition produced in accordance with the present invention may further comprise, depending on the formulation desired, a pharmaceutically acceptable, usually sterile, non-toxic carrier and/or diluent which are defined as vehicles commonly used to formulate pharmaceutical compositions for animal or human administration. The diluent is selectedso as not to affect the biological activity of the combination. Examples of such diluents are distilled water, physiological saline, Ringer's solutions, dextrose solution, and Hank's solution. In addition, the pharmaceutical composition or formulation may also include other carriers, adjuvants, or nontoxic, nontherapeutic, nonimmunogenic stabilizers and the like. Further examples of suitable pharmaceutical carriers are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. Administration of the suitable compositions may be effected by different ways, e.g., orally or parenterally by intravenous, intra-arterial, intracoronary, intraperitoneal, subcutaneous, intramuscular, topical, intradermal, transdermal, transmucosal, e.g. intranasal or intrabronchial, or by surgery or implantation (e.g., with the compound being in the form of a solid or semi-solid biologically compatible and resorbable matrix).

A therapeutically effective dose refers to that amount of compound which ameliorate the symptoms or condition. Therapeutic efficacy and toxicity of such compounds can be determined by standard pharmaceutical procedures in cell cultures or experimental animals, e.g., ED₅₀ (the dose therapeutically effective in 50% of the population) and LD₅₀ (the dose lethal to 50% of the population). The dose ratio between therapeutic and toxic effects is the therapeutic index, and it can be expressed as the ratio, LD₅₀/ED₅₀.

The dosage regimen will be determined by the attending physician and clinical factors. As is well known in the medical arts, dosages for any one patient depends upon many factors, including the patient's size, body surface area, age, the particular compound to be administered, sex, time and route of administration, general health, the individual pharmacogenetic profile and other drugs being administered concurrently. A typical dose can be, for example, in the range of 0.001 to 1000 µg (or of nucleic acid for expression of the peptide in this range); however, doses below or above this exemplary range are envisioned, especially considering the aforementioned factors. Generally, the regimen as a regular administration of the pharmaceutical composition should be in the range of 1 µg to 10 mg units per day. If the regimen is a continuous infusion, it should also be in the range of 1 µg to 10 mg units per kilogram of body weight per minute, respectively. Progress can be monitored by periodic assessment. The compositions of the invention may be administered locally or systemically.

The compositions comprising the compound which is a peptide or pharmaceutically acceptable salts thereof may conveniently be administered by any of the routes conventionally used for drug administration. Administration will generally be parenterally, e.g., intravenously or intra-arterial; DNA may also be administered directly to the target site, e.g., by biolistic delivery to an internal or external target site or by catheter to a site in an artery. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.

Acceptable salts comprise acetate, methylester, sulfate, chloride and the like. The peptide drug may be administered in conventional dosage forms prepared by combining the drugs with standard pharmaceutical carriers according to conventional procedures. The peptide drugs may also be administered in conventional dosages in combination with a known, second therapeutically active compound. Such therapeutically active compounds comprise, for example, those mentioned above. These procedures may involve mixing, granulating and compressing or dissolving the ingredients as appropriate to the desired preparation. It will be appreciated that the form and character of the pharmaceutically acceptable character or diluent is dictated by the amount of active ingredient with which it is to be combined, the route of administration and other well-known variables. The carrier(s) must be "acceptable" in the sense of being compatible with the other ingredients of the formulation and not deleterious to the recipient thereof. The pharmaceutical carrier employed may be, for example, either a solid or liquid. Exemplary of solid carriers are lactose, terra alba, sucrose, talc, gelatin, agar, pectin, acacia, magnesium stearate, stearic acid and the like. Further examples of liquid carriers are phosphate buffered saline solution, syrup, oil such as peanut oil and olive oil, water, emulsions, various types of wetting agents, sterile solutions and the like. Similarly, the carrier or diluent may include time delay material well known to the art, such as glyceryl mono-stearate or glyceryl distearate alone or with a wax.

A wide variety of pharmaceutical forms can be employed. Thus, if a solid carrier is used, the preparation can be tableted, placed in a hard gelatin capsule in powder or pellet form or in the form of a troche or lozenge. The amount of solid carrier will vary widely but preferably will be from about 25 mg to about 1 g. When a liquid carrier is used, the preparation will be in the form of a syrup, emulsion, soft gelatin capsule, sterile injectable liquid such as an ampule or nonaqueaous liquid suspension.

In a further preferred embodiment of the use of the invention, said disease is a disease associated with reduced blood flow.

In a most preferred embodiment of the use of the invention, said disease is atherosclerosis.

In a further most preferred embodiment of the use of the invention, said disease is coronary heart disease, stroke, arterial occlusive disease or an ulcer.

In a further preferred embodiment the wound is a non-infected wound.

In an even more preferred embodiment the wound is an infected wound. infection of wounds may be the result of contact with bacteria, yeasts, fungi and/or viruses. In this embodiment of the invention, a particular advantage is derived from the fact that the peptide has both an antimicrobial effect and a synergistic wound healing effect.

The skilled artisan is in a position to exchange (one or more) amino acids within the LL-37 peptide without abandoning the advantageous angiogenic effects described herein. Such exchanges would preferably be exchanges of conservative amino acids. After the exchange, the properties of the new peptide may be tested as described in the appended examples. Amino acid exchanges can be effected, for example, on the nucleic acid level by site directed mutagenesis. Exchange of amino acids can also be applied, e.g. to generate antagonists that block the interactions between LL-37 and its receptor FPRL1. This can be used according to the present invention in the treatment of cancer via the inhibition of LL-37 induced angiogenesis. Other agonists or antagonists of FPRL1 can be evaluated to identify other molecules capable in inducing angiogenesis or inhibiting angiogenesis. These other molecules could be derived from LL-37 or could be completely unrelated to LL-37.

The invention also envisages a method of refining LL-37, said method comprising the steps of (1) identification of the binding sites of the compound LL-37 and the receptor by site-directed mutagenesis or chimeric protein studies; (2) identification of the binding-site of said receptor and the compound by site-directed mutagenesis of the corresponding DNA or by chimeric protein studies, (3) molecular modeling of both the binding site of the compound and the binding site of the receptor; and (4) modification of the compound to improve its binding specificity for the receptor.

All techniques employed in the various steps of this method are conventional or can be derived by the person skilled in the art from conventional techniques without further ado. Thus, biological assays based on the herein identified nature of the peptide may be employed to assess the specificity or potency of the drugs wherein the increase of one or more activities of the peptides may be used to monitor said specificity or potency. Steps (1) and (2) can be carried out according to conventional protocols. A protocol for site directed mutagenesis is described in Ling MM, Robinson BH. (1997) Anal. Biochem. 254: 157-178. The use of homology modelling in conjunction with site-directed mutagenesis for analysis of structure-function relationships is reviewed in Szklarz and Halpert (1997) Life Sci. 61:2507-2520. Chimeric proteins are generated by ligation of the corresponding DNA fragments via a unique restriction site using the conventional cloning techniques described in Sambrook, Fritsch, Maniatis. Molecular Cloning, a laboratory manual. (1989) Cold Spring Harbor Laboratory Press. A fusion of two DNA fragments that results in a chimeric DNA fragment encoding a chimeric protein can also be generated using the gateway-system (Life technologies), a system that is based on DNA fusion by recombination. A prominent example of molecular modelling is the structure-based design of compounds binding to HIV reverse transcriptase that is reviewed in Mao, Sudbeck, Venkatachalam and Uckun (2000). Biochem. Pharmacol. 60: 1251-1265.

For example, identification of the binding site of said drug by site-directed mutagenesis and chimerical protein studies can be achieved by modifications in the receptor primary sequence that affect the peptide drug affinity; this usually allows to precisely map the binding pocket for the drug.

As regards step (2), the following protocols may be envisaged: Once the effector site for peptide drug has been mapped, the precise residues of the receptor interacting with different parts of the drug can be identified by combination of the information obtained from mutagenesis studies (step (1)) and computer simulations of the structure of the binding site provided that the precise three-dimensional structure of the peptide is known (if not, it can be predicted by computational simulation). The peptide can be also mutated to determine which residues interact with other residues in the receptor.

Finally, in step (3) the peptide can be modified to improve its binding affinity or its potency and specificity. If, for instance, there are electrostatic interactions between a particular residue of the peptide and some region of the receptor molecule, the overall charge in that region can be modified to increase that particular interaction.

Identification of binding sites may be assisted by computer programs. Thus, appropriate computer programs can be used for the identification of interactive sites of the peptide and the receptor by computer assisted searches for complementary structural motifs (Fassina, Immunomethods 5 (1994), 114-120). Further appropriate computer systems for the computer aided design of protein and peptides are described in the prior art, for example, in Berry, Biochem. Soc. Trans. 22 (1994), 1033-1036; Wodak, Ann. N. Y. Acad. Sci. 501 (1987), 1-13; Pabo, Biochemistry 25 (1986), 5987-5991. Modifications of the peptide drug can be produced, for example, by peptidomimetics and other effective compounds can also be identified by the synthesis of peptidomimetic combinatorial libraries through successive chemical modification and testing the resulting compounds. Methods for the generation and use of peptidomimetic combinatorial libraries are described in the prior art, for example in Ostresh, Methods in Enzymology 267 (1996), 220-234 and Dorner, Bioorg. Med. Chem. 4 (1996), 709-715. Furthermore, the three-dimensional and/or crystallographic structure of the peptide can be used for the design of peptidomimetic activators, e.g., in combination with the peptide (Rose, Biochemistry 35 (1996), 12933-12944; Rutenber, Bioorg. Med. Chem. 4 (1996), 1545-1558).

In accordance with the above, said peptide may be further refined by peptidomimetics.

The peptide may be further modified as a lead compound to achieve (i) modified site of action, spectrum of activity, organ specificity, and/or (ii) improved potency, and/or (iii) decreased toxicity (improved therapeutic index), and/or (iv) decreased side effects, and/or (v) modified onset of therapeutic action, duration of effect, and/or (vi) modified pharmakinetic parameters (resorption, distribution, metabolism and excretion), and/or (vii) modified physico-chemical parameters (solubility, hygroscopicity, color, taste, odor, stability, state), and/or (viii) improved general specificity, organ/tissue specificity, and/or (ix) optimized application form and route by (i) esterification of carboxyl groups, or (ii) esterification of hydroxyl groups with carbon acids, or (iii) esterification of hydroxyl groups to, e.g. phosphates, pyrophosphates or sulfates or hemi succinates, or (iv) formation of pharmaceutically acceptable salts, or (v) formation of pharmaceutically acceptable complexes, or (vi) synthesis of pharmacologically active polymers, or (vii) introduction of hydrophylic moieties, or (viii) introduction/exchange of substituents on aromates or side chains, change of substituent pattern, or (ix) modification by introduction of isosteric or bioisosteric moieties, or (x) synthesis of homologous compounds, or (xi) introduction of branched side chains, or (xii) conversion of alkyl substituents to cyclic analogues, or (xiii) derivatisation of hydroxyl group to ketales, acetales, or (xiv) N-acetylation to amides, phenylcarbamates, or (xv) synthesis of Mannich bases, imines, or (xvi) transformation of ketones or aldehydes to Schiff's bases, oximes, acetales, ketales, enolesters, oxazolidines, thiozolidines or combinations thereof.

The various steps recited above are generally known in the art. They include or rely on quantitative structure-action relationship (QSAR) analyses (Kubinyi, "Hausch-Analysis and Related Approaches", VCH Verlag, Weinheim, 1992), combinatorial biochemistry, classical chemistry and others (see, for example, Holzgrabe and Bechtold, Deutsche Apotheker Zeitung 140(8), 813-823, 2000).

A further embodiment of the invention relates to a method of treating a subject prophylactically or treating a patient which suffers from any of the above referenced diseases comprising administering a suitable amount of said pharmaceutical composition or the compound identified by the method of the invention or the refined or modified compound as described above to a patient in the need thereof.
Doses and modes of administration may be as described above. Preferably, the subject/patient is a human.

Summarizing, the results obtained in accordance with the invention implicate, inter alia, the following applications:
- Therapeutical angiogenesis in diseases with reduced or induced blood flow, such as coronary heart diseases, stroke, arterial occlusive disease.
- Stimulation of wound healing in infected and non infected wounds and ulcers. Especially the broad functional spectrum of LL-37 (angiogenic and antimicrobial) implicates it use in infected and non-infected wounds.
- Inhibition of LL-37 induced angiogenesis that contributes to tumor growth.

The figures show:

### Figure 1

Stimulation of angiogenesis by LL-37/hCAP-18 in vitro. ***a***, In vitro proliferation assays. HUVECs were seeded on six well plastic dishes and cultivated in serum free medium. Different concentrations of LL-37/hCAP-18 and control peptides were added to the culture medium. Figures on the left hand side indicate the numbers of cells per µl of volume after 72 h. VEGF was used as positive control. In vitro proliferation was not inhibited by human serum. A significance level of *P* < 0.05 is labeled with **.***b***. Preincubation of endothelial cells with pertussis toxin (PTX), wortmannin, or GF 109203X resulted in the inhibition of the proliferative response induced by LL-37/hCAP-18. ***c*** and ***d***, In vitro Matrigel assays. HUVECs were seeded on Matrigel support and cultivated in serum free medium in the absence (*c*) or presence (*d*) of 0.5 µg/ml LL-37/hCAP-18. After 18 h, the cultures were inspected for the formation of rings and cords. Representative figures are shown.

### Figure 2

Rabbit hindlimb model of arteriogenesis and angiogenesis. Application of the peptide in a rabbit hindlimb model resulted in increased collateral growth in the LL-37 treated animals *(B)* as compared to the control group *(A).* Collateral growth was quantified by counting the contrast-opacified collateral vessels in the medial thigh using a grid overlay and found a significant increase in the LL-37/hCAP-18 group (*C*). Tissue specimens from the gastrocnemius and fibularis muscles for histology revealed higher capillary density in the treated group as compared to the control group (*D*). Blood flow as assessed by cine-frame-count of the passage time of contrast agent between the common iliac artery and the anterior tibial artery was significantly augmented in the LL-37/hCAP-18 group (*E*). A significance level of *P* < 0.05 is labeled with **.

The examples illustrate the invention.

### Example 1: Detection of expression of FPRL1 in endothelial cells

Total RNA was isolated from endothelial cells from human umbilical veins HUVECs (Trizol reagent, LifeTechnologies, Karlsruhe, Germany) and reverse transcribed (Superscript II reverse transcriptase system, LifeTechnologies). Primers were designed to amplify FPRL1 cDNA based on the GenBank file (accession number NM001462): sense: 5-GAC CTT GGA TTC TTG CTC TAG TC-3'; antisense: 5'-CCA TCC TCA CAA TGC CTG TAA C-3'. Primers for glyceraldehyde-phosphate-dehydrogenase (GAPDH) were used as positive control. PCR conditions were applied as described earlier ⁷ using a RoboCycler Gradient 40 with hot top (Stratagene, Heidelberg, Germany). Products were separated by agarose gel electrophoresis, stained with ethidium bromide and viewed on a UV transilluminator. We showed by reverse transcriptase PCR that cultivated HUVECs express FPRL1. Sequencing of the 620 bp PCR product revealed the expected cDNA sequence of FPRL1.

### Example 2: In vitro Assays

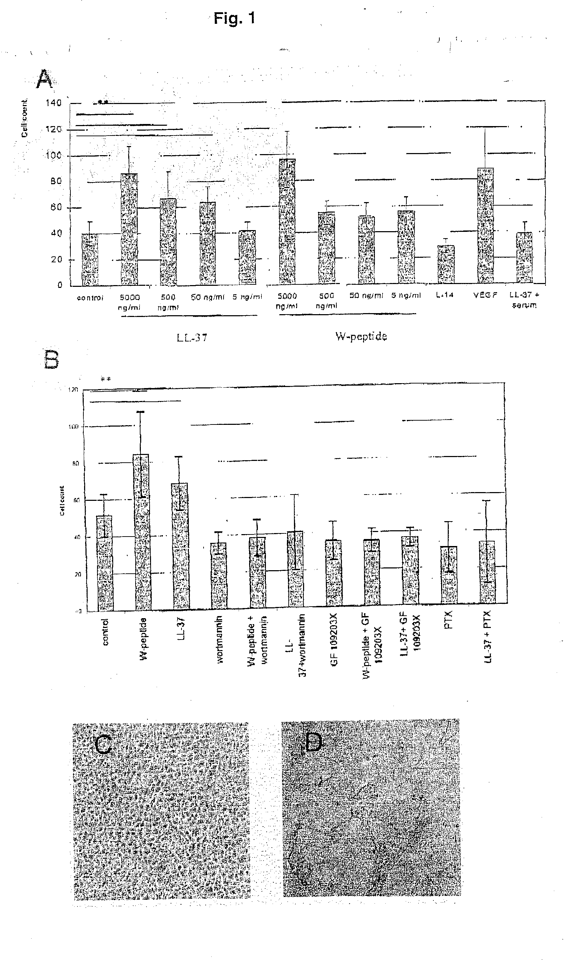

Based on these results we investigated whether agonists of FPRL1 induce reactions of endothelial cells. Endothelial cells from human umbilical veins (HUVEC) were isolated as described before ⁸. 10⁴ cells were seeded on six well plastic dishes in serum free medium. Different concentrations of LL-37 were added to the culture medium. VEGF (5 ng / ml, 165 amino acid variant, Sigma Chemicals, Munich, Germany) was used as positive control. After 72 h the cells were detached from the support and counted. Experiments were repeated ten times using synthetic LL-37/hCAP-18 (37 C-terminal amino acids) and the hexapeptide WKYMVm, wherein "m" denotes the D-form of the amino acid methionine, from two different sources (IPF Pharmaceuticals, Hannover, Germany and Institute for Biochemistry, Humboldt-University of Berlin, Germany). As control peptide we used the peptide L-14 unknown to have any interactions with FPRL1 from the same source (Humboldt-University). Human serum (10 %, donor with ABO blood group O) was applied under the same conditions to analyze any impact on induction of proliferation by LL-37. For inhibition experiments we applied inhibitors of G-protein coupled receptors (pertussis toxin, 10 ng/ml), PI-3K (wortmannin), and PKC (GF 109203X). For Matrigel assays HUVECs were seeded on Matrigel support (Beckton Dickinson, Franklin Lakes, NJ) and cultivated in serum free medium in the absence or presence of 0.5 µg/ml LL-37. After 18 h, the cultures were inspected for the formation of rings and cords. Experiments were repeated three times.
We measured the ability of LL-37/hCAP-18 to accelerate proliferation of HUVECs in a proliferation assay. Addition of the synthetic peptide to cultivated cells resulted in a dose-dependent increase of endothelial proliferation (Fig. 1 *a*). The minimal concentration of LL-37/hCAP-18 necessary to induce significant increase of proliferation was 50 ng / ml. This activity was not blocked by the presence of 10 % human serum (Fig. 1 *a*), which abolishes the antimicrobial activity of LL-37/hCAP-18, likely by binding of the peptide to apolipoprotein A-I ⁹. In addition to LL-37/hCAP-18 also the synthetic hexapeptide WKYMVm stimulated proliferation of endothelial cells (Fig. 1 *a*). WKYMVm is a specific agonist for FPRL1 ¹⁰. Next, we measured the formation of vascular structures by applying LL-37/hCAP-18 in an in vitro Matrigel assay and found that LL-37/HCAP-18 significantly induces the formation of visible rings and cords of cells (Fig. 1 *b, c*). These in vitro assays show that LL-37/hCAP-18 activates endothelial cells and induced formation of vessel like structures.

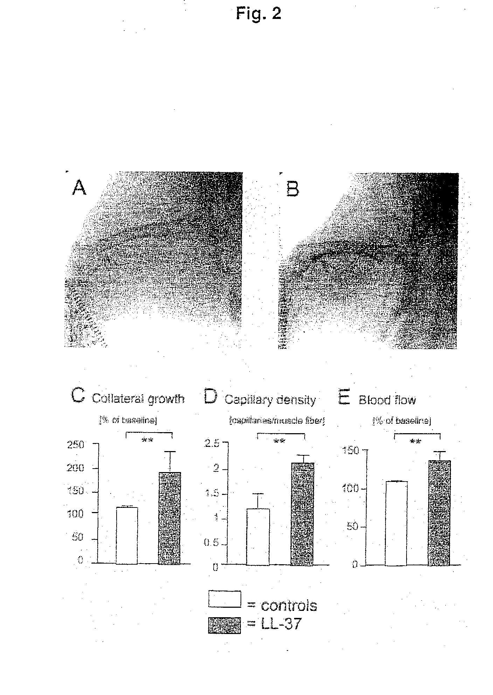

As a next step we analyzed which mechanisms might be involved in the angiogenic activity of LL-37/hCAP-18. We applied inhibitors of G-protein coupled receptors (pertussis toxin), PI-3K (wortmannin), and PKC (GF 109203X) and monitored cell proliferation as outcome parameter. While these substances had no effect on the baseline measurements, they all inhibited activities of LL-37/hCAP-18 on endothelial proliferation (Fig. 2 a).

### Example 3: Rabbit model of arteriogenesis and angiogenesis

To analyze angiogenic effects of LL-37 in vivo we used a rabbit model of angiogenesis. German Giant Rabbits (6.5 ± 0.4 kg, *n* = 8) were subjected to ligation of the external iliac artery (proximally), the popliteal and saphenous arteries (distally) and all femoral artery branches, followed by excision of the entire femoral artery 11. Seven days after surgery, synthetic LL-37 (200 µg in 1 ml PBS) or PBS alone as control were injected into the muscles of the medial thigh and the calf (5 injections sites, n = 4 / group). Seven (baseline) and thirty-five days after surgery, angiography was performed by automated injection of contrast agent into the abdominal aorta (4 ml at 2 ml / sec; cine-angiography 25 frames / sec; Siemens, Munich, Germany). Collateral growth was defined as the increase in angiographic collateral score between baseline and 35 days after initial surgery (assessed by counting the contrast-opacified collateral vessels in the medial thigh using a grid overlay) ¹². Blood flow was assessed by cine-frame-count of the passage time of contrast agent between the common iliac artery and the anterior tibial artery (proximal and distal landmarks), adapting the TIMI frame-count method to peripheral blood flow ¹³. Blood flow measurements correlate well with results obtained with doppler flow-wire in the internal iliac artery (Jomed, Haan, Germany). Tissue specimens were sampled from the right gastrocnemius and fibularis muscles for histology (alkaline phosphatase staining using an indoxyl-tetrazolium method), capillary density was assessed as the ratio of capillaries to muscle fibers (4 fields, 200 x magnitude).

To assess the effect of LL-37/hCAP-18 on both angiogenesis and the induction of collateral circulation under pathological conditions, we applied the substance in a rabbit hind limb model. In this model angiogenesis and collateral vessel growth (arteriogenesis) are induced by excision of the femoral artery. Seven days after surgical excision of the femoral artery, baseline measurements were performed and LL-37/hCAP-18 or buffer as control were injected into the ischemic hindlimb. Three weeks after injection, we found significantly increased collateral growth, blood flow in the thigh, and higher capillary density in the calf in the LL-37/hCAP-18 group as compared to the control limbs (Fig. 2 a-c). These data show that LL-37/hCAP-18 induces angiogenesis in vivo, which is accompanied by functionally increased vasculature.

### Literature

1. Buschmann, I. & Schaper, W. The pathophysiology of the collateral circulation (arteriogenesis). *J Pathol* 190, 338-42. (2000).
2. Carmeliet, P. Mechanisms of angiogenesis and arteriogenesis. *Nat Med* 6, 389-95 (2000).
3. Frohm, M. *et al*. Biochemical and antibacterial analysis of human wound and blister fluid. *Eur J Biochem* 237, 86-92. (1996).
4. Frohm, M. *et al.* The expression of the gene coding for the antibacterial peptide LL-37 is induced in human keratinocytes during inflammatory disorders. *Journal of Biological Chemistry* 272, *15258-63* (1997).
5. Bals, R., Wang, X., Zasloff, M. & Wilson, J.M. The peptide antibiotic LL-37/hCAP-18 is expressed in epithelia of the human lung where it has broad antimicrobial activity at the airway surface. *Proc Natl Acad Sci U S A* 95, 9541-6 (1998).
6. Agerberth, B. *et al.* Antibacterial components in bronchoalveolar lavage fluid from healthy individuals and sarcoidosis patients. *Am J Respir Grit Care Med* 160, 283-90. (1999).
7. Bals, R. *et al.* Human beta-defensin 2 is a salt-sensitive peptide antibiotic expressed in human lung. *J Clin Invest* 102, 874-880 (1998).
8. Zahler, S., Kupatt, C. & Becker, B.F. Endothelial preconditioning by transient oxidative stress reduces inflammatory responses of cultured endothelial cells to TNF-alpha. *Faseb J* 14, 555-64. (2000).
9. Wang, Y., Agerberth, B., Lothgren, A., Almstedt, A. & Johansson, J. Apolipoprotein A-I binds and inhibits the human antibacterial/cytotoxic peptide LL-37. *J Biol Chem* 273, 33115-8. (1998).
10. Hu, J.Y. *et al.* Synthetic peptide MMK-1 is a highly specific chemotactic agonist for leukocyte FPRL1. J *Leukoc Biol* 70,155-61. (2001).
11. Takeshita, S. *et al.* Therapeutic angiogenesis. A single intraarterial bolus of vascular endothelial growth factor augments revascularization in a rabbit ischemic hind limb model. J *Clin Invest* 93, 662-70. (1994).
12. Murohara, T. *et al.* Nitric oxide synthase modulates angiogenesis in response to tissue ischemia. *J Clin Invest* 101, 2567-78. (1998).
13. Gibson, C.M. *et al.* TIMI frame count: a quantitative method of assessing coronary artery flow. *Circulation* 93, 879-88. (1996).
14. Li, J. *et al.* PR39, a peptide regulator of angiogenesis. *Nat Med* 6, 49-55 (2000).

## Claims

1. Use of a peptide having the amino acid sequence LLGDFFRKSKEKIGKEFKRIVQRIKDFLRNLVPRTES (LL-37) for the preparation of a pharmaceutical composition for the prevention or treatment of a disease caused by or resulting in a reduced level of angiogenesis or of arteriogenesis, or for the treatment of wounds.

2. The use of claim 1 wherein said disease is a disease associated with reduced blood flow.

3. The use of claim 2 wherein said disease is atherosclerosis.

4. The use of claim 2 wherein said disease is coronary heart disease, stroke, arterial occlusive disease or an ulcer.

5. The use of claim 1 wherein said wound is an infected wound.

6. The use of claim 1 wherein said wound is a non-infected wound.

7. Use of an agent which suppresses angiogenesis through the inhibition of LL-37 for the preparation of a pharmaceutical composition for the treatment of tumors.

8. The use of claim 7, wherein the agent blocks an interaction of LL-37 and its receptor.

9. The use of claim 8, wherein the receptor is the FPRL1-receptor.

10. The use of claims 8 or 9, wherein the agent which blocks an interaction of LL-37 is
(i) an anti-LL-37 antibody or an anti-LL37-receptor antibody; and/or
(ii) a non-stimulatory form of LL-37 or a soluble form of a LL37-receptor.

11. The use of any one of claims 7 to 10, wherein the tumor is selected form the group consisting of carcinoma or sarcoma including cancer of the bile duct, brain, breast, colon, stomach, male and female reproductive organs, lung and airways, skin, gallbladder, liver, nasopharynx, nerve cells, kidney, prostate, glands and Kaposi's sarcoma.
